# EUROPEAN PATENT SPECIFICATION

(11) **EP 0 331 492 B1**
(45) Date of publication and mention of the grant of the patent: **03.07.1996**
(21) Application number: 89302090.9
(22) Date of filing: 02.03.1989
(51) Int. Cl.: C08G 75/23, A61L 2/26

(54) **Medical Devices with improved environmental stress-crack resistance made from Poly (aryl ether sulfones)**
Medizinische Erzeugnisse mit verbessertem Umweltspannungsrisswiderstand aus Polyaryläthersulfone
Articles médicaux ayant une résistance améliorée à la fissure sous-tension due à des facteurs d'environnement obtenus à partir de Polyaryléthersulfones

(30) Priority: 03.03.1988 US 163781
(43) Date of publication of application: 06.09.1989
(73) Proprietor: AMOCO CORPORATION, Chicago Illinois 60601 (US)
(72) Inventor: Sauers, Marvin Edward, Belle Mead New Jersey 08502 (US); Dickinson, Barry Lee, Whitehouse Station New Jersey 08889 (US)
(74) Representative: Dowden, Marina

(56) References cited:
- FR-A- 2 090 487
- GB-A- 1 250 470
- GB-A- 2 088 396
- EP 297 363
- Kirk-Othmer Encyclopedia of Chemical Technology, 3rd Edition, 1982, Wiley-Interscience Vol.18, Pages 832-847

## Description

### Field of the Invention

The invention is directed to the use of certain well-defined poly(arylethersulfones) in autoclavable surgical trays adapted for medical uses where transparency and excellent environmental stress-crack resistance are required.

Articles made from these poly(aryl ether sulfones) can be steam-sterilized while under stresses of 3447.4 kPa (500 psi) or greater; moreover they are not affected by corrosion-reducing additives such as morpholine, for example. Also, the above materials demonstrate good chemical resistance in contact with commonly used hospital cleaners and detergents.

### Background of the Invention

Poly(aryl ether sulfones) have been known for about two decades. They are tough linear polymers that possess a number of attractive features such as excellent high temperature resistance, good electrical properties, and very good hydrolytic stability. Two poly(aryl ether sulfones) are commercially available. A poly(aryl ether sulfone) is available from Imperial Chemical Industries, Ltd. It has the formula (1): 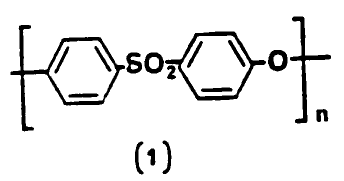 and is produced by the polycondensation of 4,4'-dihydroxydiphenyl sulfone with 4,4,-dichlorodiphenyl sulfone as described in, for example, Canadian Patent No. 847,963. The polymer contains no aliphatic moieties and has a heat deflection temperature of approximately 210°C. Another commercial poly(aryl ether sulfone) is available from Amoco Performance Products, Inc., under the trademark of UDEL® . It corresponds to formula (2), 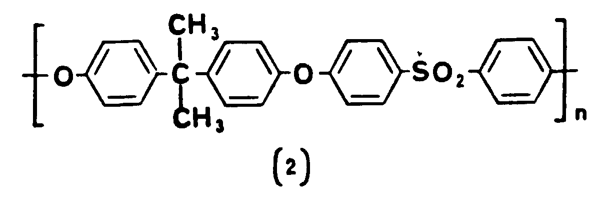 has a heat deflection temperature of about 180°C, and is made via the nucleophilic polycondensation of bisphenol-A di-sodium salt with 4,4′-dichlorodiphenyl sulfone, as described in U.S. Patent No. 4,108,837.

Over the years, there has developed a substantial body of patent and other literature directed to the formation and properties of poly(aryl ether sulfones) and other poly(aryl ethers) (all hereinafter called "PAE"). A broad range of PAE's was achieved by Johnson et al., J. of Polymer Science, A-1, Vol. 5, 1967, pp. 2415-2427; Johnson et al., U.S. Patent Nos. 4,108,837 and 4,175,175. Johnson et al. show that a very broad range of PAE's can be formed by the nucleophilic aromatic substitution (condensation) reaction of an activated aromatic dihalide and an aromatic diol. By this method, Johnson et al. created a host of new PAE's.

Because of their excellent mechanical and thermal properties, coupled with outstanding hydrolytic stability, the poly(aryl ether sulfones) have been utilized in the medical market for a variety of purposes for at least ten years. These medical devices constitute a wide variety of articles. Obviously, one of the major attributes of the poly(aryl ether sulfones) is their ability to be steam autoclaved repeatedly without loss of properties. Steam autoclaving is a very severe test, requiring both high temperature and hydrolytic stability, and involving cyclical effects - wet/dry, hot/cold.

The poly(aryl ether sulfones) (1) and (2) show some important deficiencies, however. Indeed, parts molded from these materials, stress-crack when steam sterilized under stresses of say 3447.4 kPa (500 psi) or greater; especially when boiler additives, such as morpholine are employed to reduce corrosion in the steam generating system; or, when in contact with commonly used hospital cleaners and detergents. A way, whereby these deficiencies can be circumvented is described in the present invention.

British Patent Application No. 2,088,396 describes copolymers containing units (3) and (4):

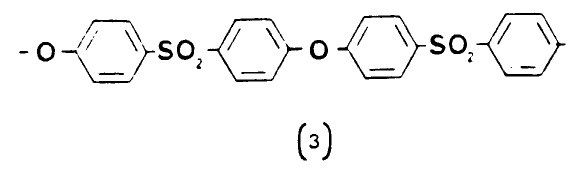

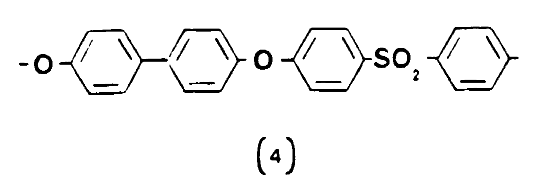

The claimed copolymers comprise about 80 to 10 mole percent of repeat units (3), and correspondingly about 20 to 90 mole percent of repeat units (4). The application states that the incorporation of (4) into the poly(aryl ether sulfone) (I) yields materials with improved resistance to hot water crazing. The application does not mention steam-sterilizability under load; nor does it teach that the copolymers show resistance to stress-cracking in the presence of boiler additives such as morpholine, or when in contact with typical hospital cleaners and detergents.

### The Invention

We have now unexpectedly discovered that certain poly(arylethersulfones) which are closely related to the materials (1) and (2), display superior stresscrack resistance when steam-sterilised under stresses of 3447.4 kPa (500 psi) or greater. These materials are unaffected by conventional corrosion-reducing additives, (e.g., morpholine, in the steam generating system, or when in contact with commonly used hospital cleaners and detergents.

The present invention, therefore, relates to the use of a poly(arylethersulfone) as defined below in an autoclavable storage tray adapted for medical use and to autoclavable storage trays made from the said poly(arylethersulfones) as defined below. The poly(arylethersulfones) used for this purpose are capable of withstanding steam sterilization at a stress level of 3447.4 kPa (500 psi). Specifically the poly(arylether sulfones) are capable of withstanding steam sterilization comprising a steam cycle conducted for 30 minutes at a temperature of 132°C (270°F) and under a pressure of 186.2 kPa (27 psi) using aqueous morpholine in the steam generating system as a corrosion reducing additive at a concentration of 50 ppm.

The poly(arylethersulfones) of the instant invention are of unit formula (5): 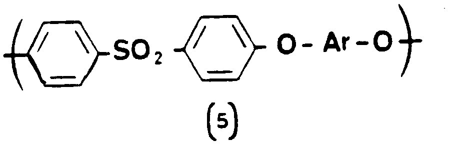 wherein Ar is said unit formula (5) is a mixture of the diradicals (6) and (8): 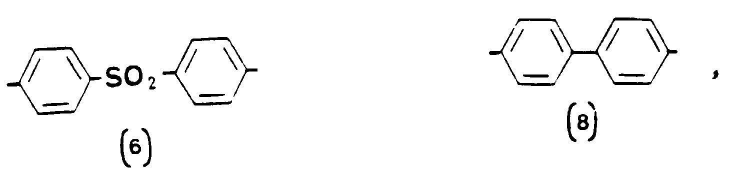 said diradical mixture(6,8), containing from 60 to 100, preferably from 75 to 100, and most preferably from 85 to 100, mole percent of the 4,4'-bidphenylene diradical (8).

In addition, blends of the poly(arylethersulfone) (5) with either of the poly(arylethersulfones) (1) and (2), defined above, wherein the weight proportion of the poly(arylethersulfone) (1) or (2) is 50 percent or less, were also found to be satisfactory for the applications hereinbefore described, i.e. for making the stress crack-resistant steam sterilizable autoclavable storage trays of the invention.

The present invention also covers the use of a poly(arylethersulfone) of unit formula (5): 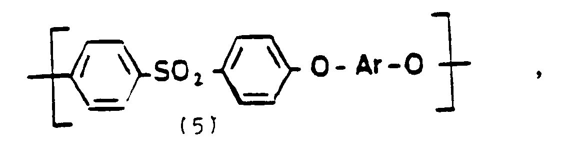 wherein Ar in said unit formula (5) is a mixture of the diradicals (6) and (8): 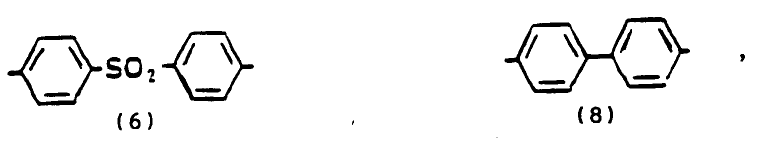 said diradical mixture (6,8) containing from 60 to 100 mole percent of the 4,4'-biphenylene diradical (8) to make a stress-crack resistant autoclavable medical storage tray of high transparency, which is steam sterilizable under stresses of 3447 kPa (500 psi) or above, in the presence of conventional corrosion reducing steam boiler additives and of conventional hospital cleaners and detergents.

The present invention further covers the use of a blend of a poly(arylethersulfone) or unit formula (5) as defined just above with a poly(arylethersulfone) of unit formula (1): 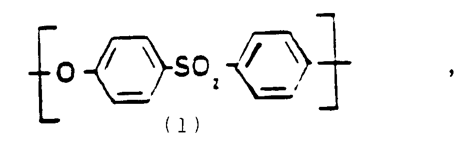 or with a poly(arylethersulfone) of unit formula (2): 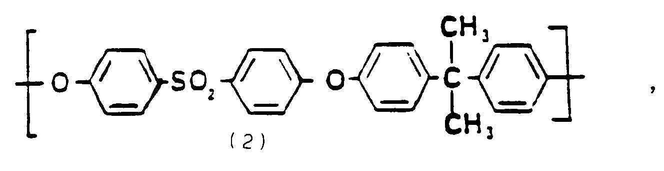 wherein the amount of said poly(arylethersulfone) (5) represents at least 50 percent by weight of said blend, to make a stress-crack resistant autoclavable medical storage tray of high transparency, which is steam sterilizable under stresses of 3447 kPa (500 psi) or above, in the presence of conventional corrosion reducing steam boiler additives and of conventional hospital cleaners and detergents.

The poly(arylethersulfones) (5) may be prepared by either of two methods, i.e., the carbonate method or the alkali metal hydroxide method.

In the carbonate method, the polymers are prepared by contacting substantially equimolar amounts of the hydroxy-containing compounds and dihalodiarylsulfones, e.g., 4,4'dichlorodiphenyl sulfone or 4,4'difluorodiphenyl sulfone, with from 0.5 to 1.0 mole of an alkali metal carbonate per mole of hydroxyl group in a solvent mixture comprising a solvent which forms an azeotrope with water in order to maintain the reaction medium at substantially anhydrous conditions during the polymerization.

The temperature of the reaction mixture is kept at 170°C to 250°C, preferably from 210°C to 235°C for one to 15 hours.

The reaction is carried out in an inert atmosphere, e.g., nitrogen, at atmospheric pressure, although higher or lower pressures may also be used.

The polyarylethersulfone is then recovered by conventional techniques such as coagulation, solvent evaporation, and the like.

The solvent mixture comprises a solvent which forms an azeotrope with water and a polar aprotic solvent. The solvent which forms an azeotrope with water includes an aromatic hydrocarbon such as benzene, toluene, xylene, ethylbenzene, and chlorobenzene.

The polar aprotic solvents employed in this invention are those generally known in the art for the manufacture of polyarylether sulfones and include sulfur containing solvents such as those of the formula:

R₁-S(O)_{b}-R₁

in which each R₁ represents a monovalent lower hydrocarbon group free of aliphatic unsaturation, which preferably contains less than 8 carbon atoms or when connected together represents a divalent alkylene group with b being an integer from 1 to 2 inclusive. Thus, in all of these solvents, all oxygens and two carbon atoms are bonded to the sulfur atom. Contemplated for use in this invention are such solvents as those having the formula: 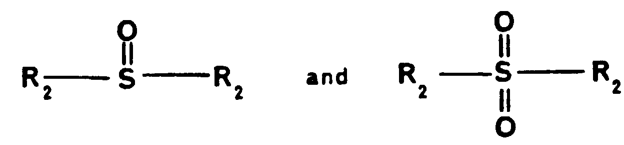 where the R₂ groups are independently lower alkyl, such as methyl, ethyl, propyl, butyl, and like groups, and aryl groups such as phenyl and alkylphenyl groups such as the tolyl group, as well as those where the R₂ groups are interconnected as in a divalent alkylene bridge such as 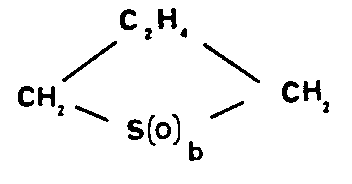 in tetrahydrothiophene oxides and dioxides. Specifically, these solvents include dimethylsulfoxide, dimethylsulfone, diphenylsulfone, diethylsulfoxide, diethylsulfone, diisopropylsulfone, tetrahydrothiophene 1,1-dioxide (commonly called tetramethylene sulfone or sulfolane) and tetrahydrothiophene-1 monoxide.

Additionally, nitrogen containing solvents may be used. These include dimethylacetamide, dimethylformamide and N-methylpyrrolidone.

The azeotrope forming solvent and polar aprotic solvent are used in a weight ratio of from 1:10 to 1:1, preferably from 1:5 to 1:3.

In the reaction, the hydroxy containing compound is slowly converted, in situ, to the alkali salt thereof by reacting with the alkali metal carbonate. The alkali metal carbonate is preferably potassium carbonate. As indicated before, mixtures of carbonates such as potassium and sodium carbonate may also be used.

Water is continuously removed from the reaction mass as an azeotrope with the azeotrope forming solvent so that substantially anhydrous conditions are maintained during the polymerization.

It is essential that the reaction medium be maintained substantially anhydrous during the polycondensation. While amounts of water up to about one percent can be tolerated, and are somewhat beneficial when employed with fluorinated dihalobenzenoid compounds, amounts of water substantially greater than this are desirably avoided as the reaction of water with the halo and/or nitro compound leads to formation of phenolic species and only low molecular weight products are secured. Consequently, in order to secure the high polymers, the system should be substantially anhydrous, and preferably contain less that 0.5 percent by weight water during the reaction.

Preferably, after the desired molecular weight has been attained, the polymer is treated with an activated aromatic halide or an aliphatic halide such as methyl chloride or benzyl chloride, and the like. Such treatment of the polymer converts the terminal hydroxyl groups into ether groups which stabilize the polymer. The polymer so treated has good melt and oxidative stability.

While the carbonate method for preparing the polymer employed according to this invention is simple and convenient, in some cases products of higher molecular weight can be made by the alkali metal hydroxide method. In the alkali metal hydroxide method, described by Johnson et al., U. S. Patent Nos. 4,108,837 and 4,175,175, a double alkali metal salt of a dihydric phenol is contacted with a dihalobenzenoid compound in the presence of a sulfur containing solvent as herein above defined under substantially anhydrous conditions.

Additionally, the polymers employed in this invention may be prepared by other methods known in the prior art, in which at least one dihydric phenol and at least one dihalobenzenoid compound are heated, for example, with a mixture of sodium carbonate or bicarbonate and a second alkali metal carbonate or bicarbonate having a higher atomic number than that of sodium, as described in U. S. Patent No. 4,176,222.

The molecular weight of the poly(aryl ethers) utilized for manufacturing the devices of the instant invention is indicated by reduced viscosity data in an appropriate solvent such as methylene chloride, chloroform and N,methylpyrrolidone. The reduced viscosities of the materials, as measured at concentrations of 0.2g per 100 ml. at 25°C, are at least 0.3 dl/g, preferably at least 0.4 dl/g and, typically, not exceeding about 1.5 dl/g.

The poly(aryl ether sulfones) employed in the instant invention allow for the fabrication of medical devices having excellent transparency as well as outstanding stress-crack resistance. These devices can be steam-sterilized under stresses of 3447.4 kPa (500 psi) or greater and in the presence of a variety of steam boiler additives. Typical boiler additives designed to reduce corrosion in steam generating systems are amino compounds such as morpholine, hydrazine, N,N-diethylaminoethanol ("NALCO 359" or "BETZ NA-9"), and octadecylamine. Steam sterilization is also possible in the presence of various hospital cleaners and detergents, such as those sold under the trademanes of "Castle 7900" (a sonic cleaner), "Chem Crest 14" (an ultrasonic cleaner), "Tergitol Min Foam 2X" (a non ionic surfactant), and the like.

The materials employed in the instant invention may include pigments, thermal stabilizers, ultraviolet light stabilizers, and other additives.

### EXAMPLES

The following examples serve to give specific illustration of the practice of this invention.

### Materials

**Polysulfone UDEL P-1700** - a poly(aryl ether sulfone) of formula (2), melt-flow at 343°C (650°F) and 303.4 kPa (44 psi) of 6.5 g/10 minutes. Sold by Amoco Performance Products, Inc.
**Polysulfone I** - a poly(aryl ether sulfone) of formula (5), wherein Ar is a mixture of (6) and (7) in a mole ratio 25:75. and having a reduced viscosity from 0.5 to 0.55 as measured in N-methylpyrrolidone, at 25°C, at a concentration of 0.2 g/100 ml. 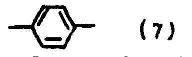
**Polysulfone II** - a poly(aryl ether sulfone) of the formula: 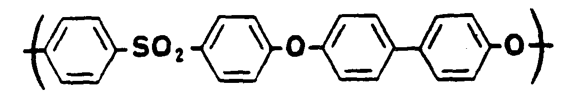 having a reduced viscosity from 0.5 to 0.55 as measured in N-methylpyrrolidone, at 25°C, at a concentration of 0.2 g/100 ml.
   A blend (60:40 by weight) of polysulfones I/UDEL P-1700 was also prepared and used in the steam sterilization experiments.

### Comparative Example A and Examples 1-3

### Procedure

The materials to be tested were molded into 3.175 mm (0.125 inch) flex bars 127 mm by 12.7 mm [(5 inch by 0.5 inch)]. These bars were autoclaved under constant stress applied via the weighted cantilever beam method. The experiments were performed in a Pelton and Crane tabletop autoclave with a 50 ppm aqueous morpholine solution. Each cycle comprised a steam cycle of 30 minutes at 132°C (270°F) under 186.2 kPa (27 psi), followed by a cooling cycle of a minimum of 10 minutes at room temperature. The cooling samples were examined for the presence of any defects, cracks, breakages, etc.

The results, summarized in Table I, clearly show that the poly(aryl ether sulfones) of the instant invention display steam sterilizability under stresses of 3447.4 kPa (500 psi) or greater, in the presence of morpholine. It is noteworthy that even the Polysulfone I/UDEL P-1700 60:40 (by weight) blend is significantly better than UDEL P-1700 under a 6894.8 kPa (1000 psi) stress; and is far superior to UDEL P-1700 under a stress of 3447. 4 kPa (500 psi). The discrepancies noted in Table I are probably due to variations in blending efficiency and, hence, quality of the resulting materials.

**TABLE I**

| Steam Sterilization Using a 50 ppm Aqueous Morpholine Solution | | | |
|---|---|---|---|
| Example | Poly(Aryl Ether Sulfone) | Stress(psi) kPa | Number of Cycles |
| Comparative A | UDEL P-1700 | 6894.8 (1000) | 79 (fail) |
| | | 3447.4 (500) | 148 (fail) |
| | | 0 (0) | 400 |
| | | | |
| 1 | Blend Polysulfone I/ | 6894.8 (1000) | 107 (fail) |
| | UDEL P-700 (60:40, | 3447.4 (500) | 476 (fail) |
| | by weight) | 0 (0) | 1012 |
| | | | |
| | | 6894.8 (1000) | 185 (fail) |
| | | 3447.4 (500) | 1005 (fail) |
| | | 0 (0) | 1012 |
| | | | |
| 2 | Polysulfone I | 6894.8 (1000) | 272 (fail) |
| | | 3447.4 (500) | 1048 |
| | | 0 (0) | 1048 |
| | | | |
| 3 | Polysulfone II | 6894.8 (1000) | 1320 |
| | | 3447.4 (500) | 1320 |
| | | 0 (0) | 1320 |

### Comparative Example B and Example 4

To determine the effect of cleaners and detergents, a test was run at 82°C (180°F) for 24 hours using 3.175 mm (1/8 inch) bars made from UDEL P-1700 (Comparative Example B) and from the other poly(aryl ether sulfones). Aqueous solutions of various cleaners and detergents were utilized. The stress was applied by bending the bar (constant strain). Note that the stress values were calculated for room temperature; therefore, their actual values may be lower at 99°C (180°F). The data are summarized in Table II. The data of Table II clearly show that Polysulfone I and Polysulfone II performed much better than UDEL P-1700.

**TABLE II**

| Effect of Various Cleaners and Detergents | | | | |
|---|---|---|---|---|
| Reagent | Stress kPa Level (psi) | UDEL p-1700 | Polysulfone I | Polysulfone II |
| Castle 7900 | 0 (0) | Surface Cracks | OK | OK |
| Sonic Cleaner | 6894.8 (1000) | TD*Cracks | OK | OK |
| (2 oz/gal) | 13789.5 (2000) | Rupture | OK | OK |
| | 27579.0 (4000) | Rupture | OK | OK |
| | | | | |
| Chem Crest 14 | 0 (0) | OK | OK | OK |
| Ultrasonic | 6894.8 (1000) | TD*Cracks | OK | OK |
| Cleaner | 13789.5 (2000) | Rupture | OK | OK |
| (2 oz/gal) | 27579.0 (4000) | Rupture | OK | OK |
| | | | | |
| Tergitol Min | 0 (0) | OK | OK | OK |
| Foam 2X | 6894.8 (1000) | Rupture | OK | OK |
| Nonionic | 13789.5 (2000) | Rupture | OK | OK |
| Surfactant** (5 ml/liter) | 27579.0 (4000) | Rupture | Rupture | OK |

| | | | | |
|---|---|---|---|---|
| * TD = transverse direction | | | | |
| ** Produced by Union Carbide Corporation. The formula is: C₁₂₋₁₄H₂₅₋₂₉O(CH₂CH₂O)ₓ[CH₂CH₂O/CH₂CH(CH₃)O]_{y}CH₂CH(CH₃)OH; Molecular weight is about 640; and x and y are 1 or greater. | | | | |

## Claims

1. A stress-crack resistant autoclavable medical storage tray of high transparency, steam sterilizable under stresses of 3447 kPa (500 psi) or above, in the presence of conventional corrosion reducing steam boiler additives and of various conventional hospital cleaners and detergents, said autoclavable medical storage tray being composed of a poly(arylethersulfone) of unit formula (5): 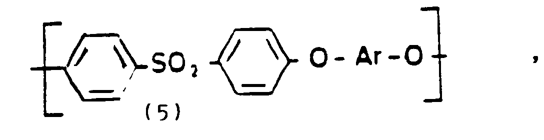 wherin Ar in said unit formula (5) is a mixture of the diradicals (6) and (8): 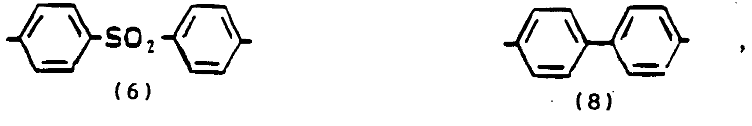 said diradical mixture (6,8) containing from 60 to 100 mole percent of the 4,4'-biphenylene diradical (8).

2. An autoclavable medical storage tray composed of a blend of a poly(arylethersulfone) of unit formula (5), as defined in Claim 1, with a poly(arylethersulfone) of unit formula (1): 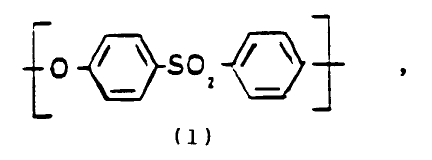 or with a poly(arylethersulfone) of unit formula (2): 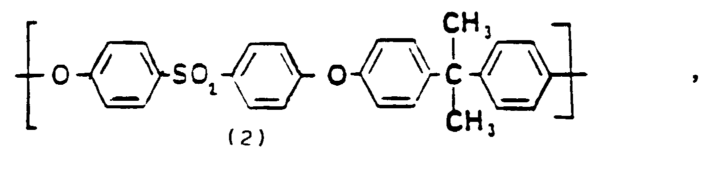 wherein the amount of said poly(arylethersulfone) (5) represents at least 50 percent by weight of said blend.

3. An autoclavable medical storage tray as defined in Claim 1 or 2, wherein said poly(arylethersulfone) (5) has a reduced viscosity of from 0.3 to 1.5 dl/g, as measured in methylene chloride or in chloroform, at a concentration of 0.2g per 100 ml, and at 25°C.

4. An autoclavable medical storage tray according to any of Claims 1-3, wherein the group Ar in said unit formula (5), as defined in Claim 1, is a mixture of said diradicals (6) and (8), as defined in Claim 1, containing from 75 to 100 mole percent of said radical (8).

5. An autoclavable medical storage tray according to Claim 4, wherein said diradical mixture (6,8) contains from 85 to 100 mole percent of said radical (8).

6. An autoclavable medical storage tray according to Claim 5, wherein said poly(arylethersulfone) (5) has the unit formula (5-1 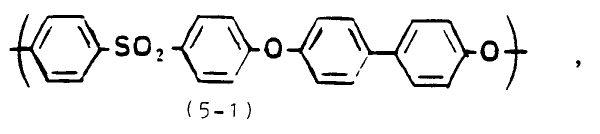 and said poly(arylethersulfone) (5-1) has a reduced viscosity of from 0.5 to 0.55, as measured in N-methylpyrrolidone at 25°C and at a concentration of 0.2 g/100 ml.

7. The use of a poly(arylethersulfone) of unit formula (5): 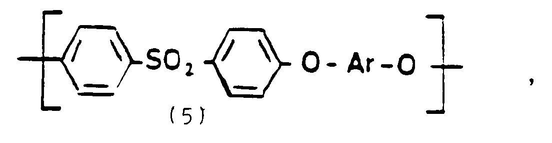 wherein Ar in said unit formula (5) is a mixture of the diradicals (6) and (8): 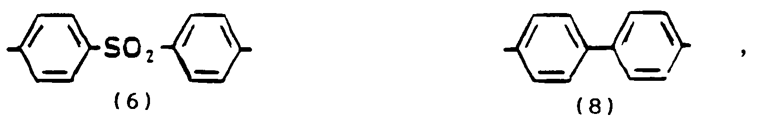 said diradical mixture (6,8) containing from 60 to 100 mole percent of the 4,4'-biphenylene diradical (8) to make a stress-crack resistant autoclavable medical storage tray of high transparency, which is steam sterilizable under stresses of 3447 kPa (500 psi) or above, in the presence of conventional corrosion reducing steam boiler additives and of conventional hospital cleaners and detergents.

8. The use of a blend of a poly(arylethersulfone) of unit formula (5), as defined in Claim 7, with a poly(arylethersulfone) of unit formula (1); 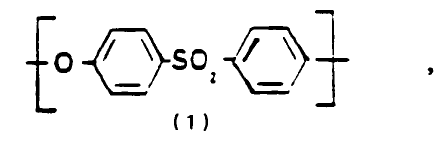 or with a poly(arylethersulfone) of unit formula (2): 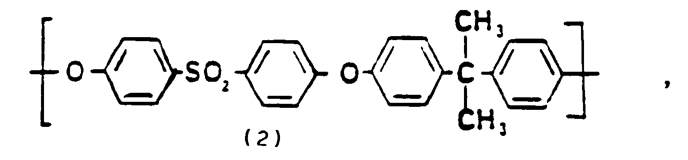 wherein the amount of said poly(arylethersulfone) (5) represents at least 50 percent by weight of said blend, to make a stress-crack resistant autoclavable medical storage tray of high transparency, which is steam sterilizable under stresses of 3447 kPa (500 psi) or above, in the presence of conventional corrosion reducing steam boiler additives and of conventional hospital cleaners and detergents.

## Patentansprüche

1. Medizinischer Aufbewahrungsbehälter von hoher Transparenz und Spannungsrißwiderstand, der in einem Autoklaven verwendet werden kann und unter Belastung von 3447 kPa (500 psi) oder darüber Dampf-sterilisierbar ist, und zwar in Anwesenheit eines herkömmlichen, die Korrosion reduzierenden Dampfkessel-Additivs und von verschiedenen herkömmlichen, im Krankenhaus verwendeten Reinigungsmitteln und Detergentien, wobei der genannte, in einem Autoklaven verwendbare medizinische Aufbewahrungsbehälter aus einem Poly(arylethersulfon) der Formeleinheit (5) besteht: 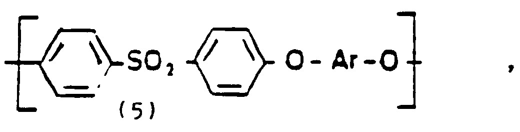 worin Ar in der Formeleinheit (5) eine Mischung der zweiwertigen Gruppe (6) und (8) ist: 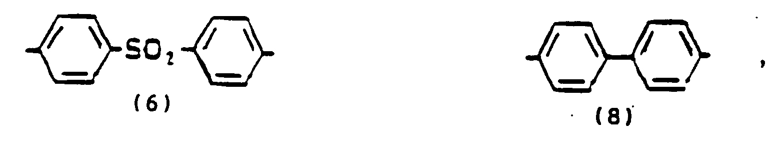 und wobei die genannte Mischung aus zweiwertigen Gruppen (6,8) von 60 bis 100 Mol-% der zweiwertigen 4,4'-Biphenylen-Gruppe (8) enthält.

2. In einem Autoklaven verwendbarer medizinischer Aufbewahrungsbehälter, bestehend aus einer Mischung eines Poly(arylethersulfons) der Formeleinheit (5) nach Anspruch 1 mit einem Poly(arylethersulfon) der Formeleinheit (1): 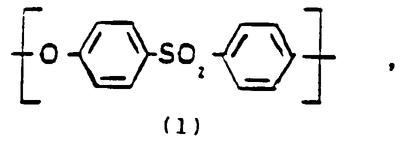 oder mit einem Poly(arylethersulfon) der Formeleinheit (2): 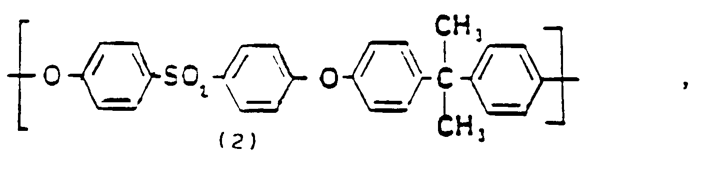 worin die Menge des genannten Poly(arylethersulfons) (5) wenigstens 50 Gew.-% der genannten Mischung ausmacht.

3. In einem Autoklaven verwendbarer medizinischer Aufbewahrungsbehälter nach Anspruch 1 oder 2, worin das genannte Poly(arylethersulfon) (5) eine reduzierte Viskosität von 0,3 bis 1,5 dl/g aufweist, bestimmt in Methylenchlorid oder in Chloroform, in einer Konzentration von 0,2 g pro 100 ml und bei 25° C.

4. In einem Autoklaven verwendbarer medizinischer Aufbewahrungsbehälter nach einem der Ansprüche 1-3, worin die Gruppe Ar in der genannten Formeleinheit (5) nach Anspruch 1 eine Mischung der genannten zweiwertigen Gruppen (6) und (8) nach Anspruch 1 ist und von 75 bis 100 Mol-% der genannten Gruppe (8) enthält.

5. In einem Autoklaven verwendbarer medizinischer Aufbewahrungsbehälter nach Anspruch 4, wobei die genannte Mischung der zweiwertigen Gruppen (6,8) von 85 bis 100 Mol-% der genannten Gruppe (8) enthält.

6. In einem Autoklaven verwendbarer medizinischer Aufbewahrungsbehälter nach Anspruch 5, wobei das genannte Poly(aryl-ethersulfon) (5) die Formeleinheit (5-1) hat: 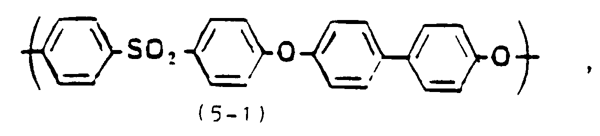 und das genannte Poly(arylethersulfon) (5-1) eine reduzierte Viskosität von 0,5 bis 0,55, gemessen in N-Methylpyrrolidon bei 25° C und einer Konzentration von 0,2 g/100 ml, aufweist.

7. Verwendung eines Poly(arylethersulfons) der Formeleinheit (5): 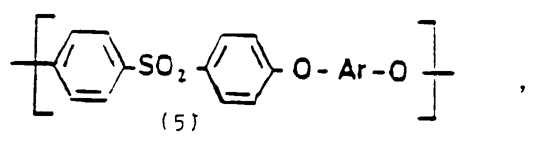 worin Ar in der genannten Formeleinheit (5) eine Mischung der zweiwertigen Gruppen (6) und (8) ist: 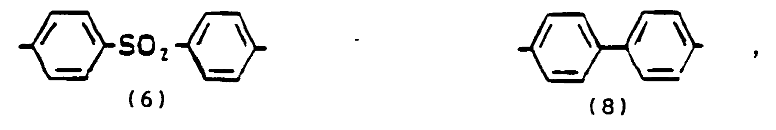 und die genannte Mischung der zweiwertigen Gruppen (6,8) von 60 bis 100 Mol-% der zweiwertigen 4,4'-Biphenylen-Gruppe (8) enthält, um einen medizinischen Aufbewahrungsbehälter von hoher Transparenz und Spannungsrißwiderstand herzustellen der in einem Autoklaven verwendet werden kann und unter Belastung von 3447 kPA (500 psi) oder mehr Dampf-sterilisierbar ist, und zwar in Anwesenheit eines herkömmlichen, die Korrosion reduzierenden Dampfkessel-Additivs und von verschiedenen herkömmlichen, im Krankenhaus verwendeten Reinigungsmitteln und Detergentien.

8. Verwendung einer Mischung eines Poly(arylethersulfons) der Formeleinheit (5) nach Anspruch 7 mit einem Poly(arylethersulfon) der Formeleinheit (1): 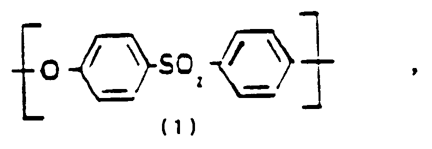 oder mit einem Poly(arylethersulfon) der Formeleinheit (2): 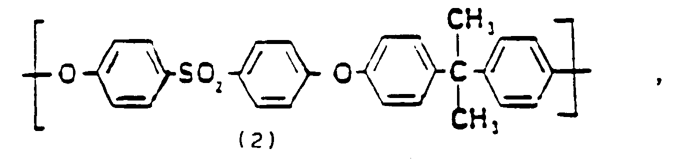 worin die Menge des genannten Poly(arylethersulfons) (5) wenigstens 50 Gew.-% der genannten Mischung ausmacht, um einen medizinischen Aufbewahrungsbehälter von hoher Transparenz und Spannungsrißwiderstand herzustellen, der in einem Autoklaven verwendet werden kann und unter Belastung von 3447 kPA (500 psi) oder mehr Dampf-sterilisierbar ist, und zwar in Anwesenheit eines herkömmlichen, die Korrosion reduzierenden Dampfkessel-Additivs und von verschiedenen herkömmlichen, im Krankenhaus verwendeten Reinigungsmitteln und Detergentien.

## Revendications

1. Plateau de stockage à usage médical autoclavable, résistant à la fissure sous contrainte, de transparence élevée, stérilisable à la vapeur d'eau sous des contraintes de 3447 kPa (500 psi) ou plus, en présence d'additifs classiques de chaudière à vapeur d'eau réduisant la corrosion et de plusieurs nettoyants et détergents hospitaliers classiques, ledit plateau de stockage à usage médical autoclavable étant composé d'une poly(aryléthersulfone) de formule de motif (5): 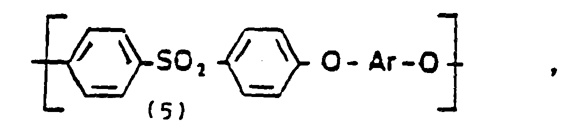 dans laquelle Ar dans ladite formule de motif (5) est un mélange des diradicaux (6) et (8) 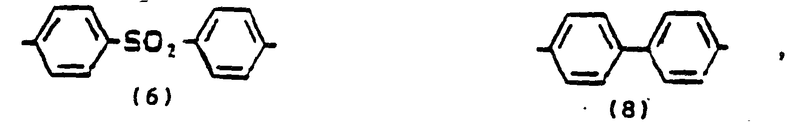 ledit mélange de diradicaux (6,8) contenant 60 à 100 pourcent en moles du diradical 4,4'-biphénylène (8).

2. Plateau de stockage à usage médical autoclavable composé d'un mélange de poly(aryléthersulfone) de formule de motif (5), tel que défini dans la revendication 1, avec une poly(aryléthersulfone) de formule de motif (1): 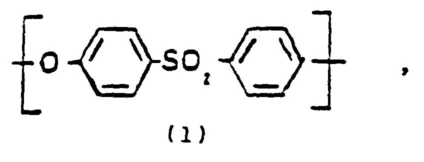 ou avec une poly(aryléthersulfone) de formule de motif (2): 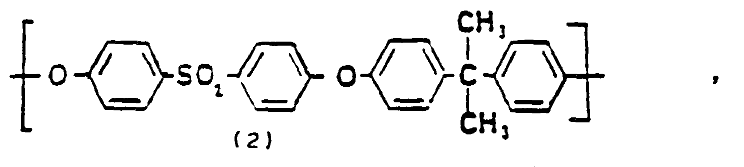 dans laquelle la quantité de ladite poly(aryléthersulfone) (5) représente au moins 50 pourcent en poids dudit mélange.

3. Plateau de stockage à usage médical autoclavable tel que défini dans la revendication 1 ou 2, dans lequel ladite poly(aryléthersulfone) (5) possède une viscosité réduite comprise entre 0,3 et 1,5 dl/g, telle que mesurée dans le chlorure de méthylène ou dans le chloroforme, à une concentration de 0,2 g pour 100 ml, et à 25°C.

4. Plateau de stockage à usage médical autoclavable selon l'une quelconque des revendications 1 à 3, dans lequel le groupe Ar dans ladite formule de motif (5), tel que défini dans la revendication 1, est un mélange desdits diradicaux (6) et (8) tel que défini dans la revendication 1, contenant de 75 à 100 pourcent en moles dudit radical (8).

5. Plateau de stockage à usage médical autoclavable selon la revendication 4, dans lequel ledit mélange de diradicaux (6,8) contient de 85 à 100 pourcent en moles dudit radical (8).

6. Plateau de stockage à usage médical autoclavable selon la revendication 5, dans lequel ladite poly(aryléthersulfone) (5) possède la formule de motif (5-1) 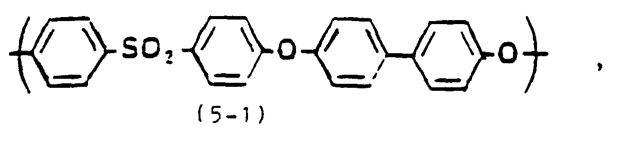 et ladite poly(aryléthersulfone) (5-1) possède une viscosité réduite comprise entre 0,5 et 0,55 dl/g, telle que mesurée dans la N-méthylpyrrolidone à une température de 25°C et à une concentration de 0,2 g/100 ml.

7. Utilisation d'une poly(aryléthersulfone) de formule de motif (5): 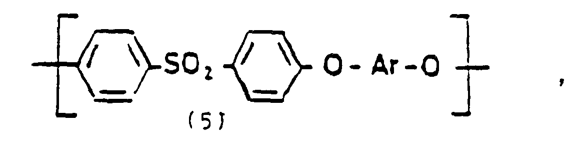 dans laquelle Ar dans ladite formule de motif (5) est un mélange des diradicaux (6) et (8) 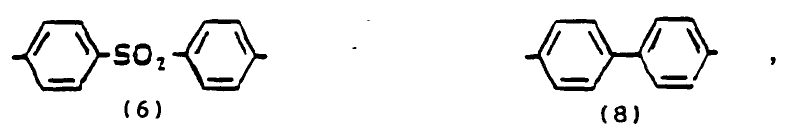 ledit mélange de diradicaux (6,8) contenant 60 à 100 pourcent en moles du diradical 4,4'-biphénylène (8) pour fabriquer un plateau de stockage à usage médical autoclavable, résistant à la fissure sous contrainte, de transparence élevée, qui est stérilisable à la vapeur d'eau sous des contraintes de 3447 kPa (500 psi) ou supérieures, en présence d'additifs classiques de chaudière à vapeur d'eau réduisant la corrosion et de nettoyants et détergents hospitaliers classiques.

8. Utilisation d'un mélange de poly(aryléthersulfone) de formule de motif (5), comme défini dans la revendication 7, avec une poly(aryléthersulfone) de formule de motif (1); 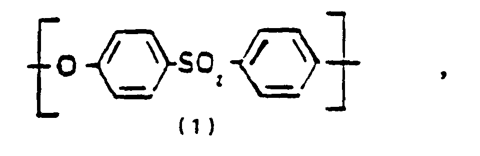 ou avec une poly(aryléthersulfone) de formule de motif (2) 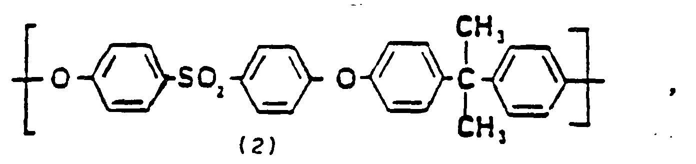 dans laquelle la quantité de ladite poly(aryléthersulfone) (5) représente au moins 50 pourcent en poids dudit mélange, pour fabriquer un plateau de stockage à usage médical autoclavable, résistant à la fissure sous contrainte, de transparence élevée, qui est stérilisable à la vapeur d'eau sous des contraintes de 3447 kPa (500 psi) ou supérieures, en présence d'additifs classiques de chaudière à vapeur d'eau réduisant la corrosion et de nettoyants et détergents hospitaliers classiques.
